(19) Europäisches Patentamt
European Patent Office
Office européen des brevets

(11) Veröffentlichungsnummer : **0 173 153**
**B1**

(12) # EUROPÄISCHE PATENTSCHRIFT

(45) Veröffentlichungstag der Patentschrift : **01.02.89**

(51) Int. Cl.[4] : **C 07 D319/20**, C 07 D319/22

(21) Anmeldenummer : **85110065.1**

(22) Anmeldetag : **10.08.85**

(54) **Neue benzokondensierte, fluorierte, heterocyclische Verbindungen, ein Verfahren zu ihrer Herstellung und ihre Verwendung.**

(30) Priorität : **24.08.84 DE 3431222**

(43) Veröffentlichungstag der Anmeldung : **05.03.86 Patentblatt 86/10**

(45) Bekanntmachung des Hinweises auf die Patenterteilung : **01.02.89 Patentblatt 89/05**

(84) Benannte Vertragsstaaten : **BE CH DE FR GB IT LI NL**

(56) Entgegenhaltungen :
**EP-A- 0 041 131**
**EP-A- 0 042 533**
**EP-A- 0 093 977**
**EP-A- 0 097 862**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Marhold, Albrecht, Dr.**
**Carl-Duisberg-Strasse 329**
**D-5090 Leverkusen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 173 153 B1

**Beschreibung**

Benzodioxene, die im Dioxenteil mit Fluor und gegebenenfalls zusätzlich mit Chlor substituiert sind, sind aus EP-A 0 041 131, EP-A 0 042 533, EP-A 0 093 977 und EP-A 0 097 862 bekannt. Diese können jedoch die erfindungsgemäßen Benzodioxene nicht nahelegen, da erfindungsgemäße Benzodioxene mindestens einen über eine C-C-Bindung verknüpften Substituenten aufweisen. C-Halogen- und C-H-Bindungen können die Existenz entsprechender C-C-Bindungen nicht nahelegen. Außerdem sind erfindungsgemäße Verbindungen nur auf grundsätzlich andere Weise herstellbar als die bekannten Verbindungen.

Die vorliegende Erfindung betrifft neue benzokondensierte, fluorierte, heterocyclische Verbindungen der Formel

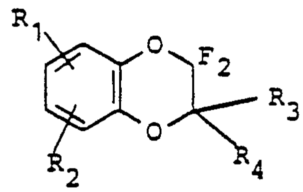

(I)

in der $R_1$ und $R_2$ unabhängig voneinander für H, F, Cl, $CH_3$, $NO_2$, CO—O—$C_1$- bis $C_4$-Alkyl, COCl oder Phenyl oder $R_1$ und $R_2$ gemeinsam für 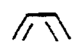 , $R_3$ für H, $C_1$- bis $C_4$-Alkyl, CO—O—$C_1$—$C_4$-Alkyl, CN oder Phenyl und $R_4$ für $C_1$- bis $C_4$-Alkyl oder Phenyl stehen.

Besonders bevorzugt stehen in Formel (I) $R_1$ für H, $R_2$ für H, $NO_2$, $CH_3$, CO—O—$C_1$- bis $C_4$-Alkyl, COCl oder Phenyl oder $R_1$ und $R_2$ gemeinsam für 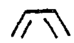 , $R_3$ für H, $CH_3$, CO—O—$CH_3$, CN oder Phenyl und $R_4$ für $CH_3$ oder Phenyl.

Das erfindungsgemäße Verfahren zur Herstellung der neuen chemischen Verbindungen der Formel (I)

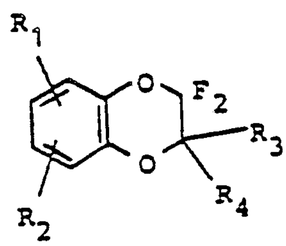

(I)

in der $R_1$ und $R_2$ unabhängig voneinander für H, F, Cl, $CH_3$, $NO_2$, CO—O—$C_1$- bis $C_4$-Alkyl, COCl oder Phenyl oder $R_1$ und $R_2$ gemeinsam für 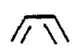 , $R_3$ für H, $C_1$- bis $C_4$-Alkyl, CO—O—$C_1$—$C_4$-Alkyl, CN oder Phenyl und $R_4$ für $C_1$- bis $C_4$-Alkyl oder Phenyl stehen, ist dadurch gekennzeichnet, daß man

a) Brenzkatechin oder ein Brenzkatechinderivat der Formel (II)

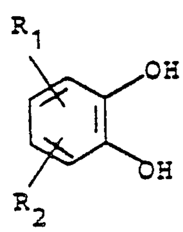

(II)

in der $R_1$ und $R_2$ die oben angegebene Bedeutung haben, mit Verbindungen der Formel (III)

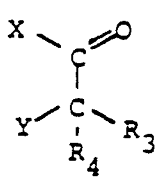

(III)

in der

$R_3$ und $R_4$ die oben angegebene Bedeutung haben,
X für Cl, Br, O—$C_1$- bis $C_4$-Alkyl oder S—$C_1$- bis $C_4$-Alkyl und
Y für Cl oder Br steht,

in Gegenwart eines Lösungsmittels und unter Zusatz eines Kondensationsmittels zu Verbindungen der Formel (IV),

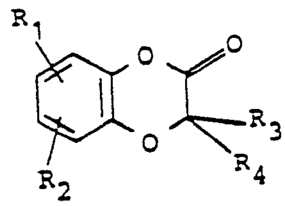

(IV)

in der $R_1$, $R_2$, $R_3$ und $R_4$ die oben angegebene Bedeutung haben, umsetzt,

b) die Verbindungen der Formel (IV) bei erhöhter Temperatur und mindestens mit einer äquimolaren Menge Phosphorpentachlorid zu Verbindungen der Formel (V)

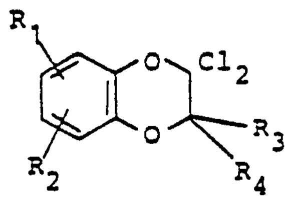

(V)

in der $R_1$, $R_2$, $R_3$ und $R_4$ die oben angegebene Bedeutung haben, umsetzt und

c) die Verbindungen der Formel (V) mit einem Fluorierungsmittel zu Verbindungen der Formel (I) umsetzt.

Besonders bevorzugt werden Verbindungen der Formel (II) eingesetzt, bei denen $R_1$ für H, $R_2$ für H, $CH_3$, $NO_2$, CO—O—$C_1$- bis $C_4$-Alkyl, COCl oder Phenyl oder $R_1$ und $R_2$ gemeinsam für 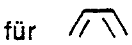 stehen.

Sofern in Formel (III) X für O—$C_1$- bis $C_4$-Alkyl oder S—$C_1$- bis $C_4$-Alkyl steht, sind als Alkylreste Methylreste bevorzugt.

In Formel (III) steht $R_3$ vorzugsweise für H, $CH_3$, CO—O—$CH_3$, CN oder Phenyl und $R_4$ vorzugsweise für $CH_3$ oder Phenyl.

Die Verbindungen der Formel (II) sind entweder Handelsprodukte oder auf einfache Weise zugänglich, beispielsweise gemäß Houben-Weyl, Methoden der organischen Chemie, Band VI/1c, Seite 327 (1976) oder auf dazu analoge Weise.

Die Verbindungen der Formel (III) sind entweder Handelsprodukte oder auf einfache Weise zugänglich, beispielsweise gemäß US-PS 2 778 851 oder auf dazu analoge Weise.

Bezogen auf 1 Mol einer Verbindung der Formel (II) können beispielsweise 1 bis 1,5 Mole einer Verbindung der Formel (III) eingesetzt werden. Vorzugsweise werden auf 1 Mol einer Verbindung der Formel (II) 1 bis 1,2 Mole einer Verbindung der Formel (III) eingesetzt.

Der Reaktionsschrit a) wird in Gegenwart eines Lösungsmittels und unter Zusatz eines Kondensationsmittels durchgeführt. Geeignete Lösungsmittel sind beispielsweise Wasser, Alkohole, z. B. solche mit 1 bis 4 C-Atomen, Acetonitril, Dimethylformamid oder Mischungen davon. Geeignete Kondensationsmittel sind beispielsweise basische Verbindungen, wie Alkali- oder Erdalkalihydroxide, Alkali- oder Erdalkalicarbonate oder Amine, wie Pyridin und N,N-Dimethylanilin oder Mischungen davon. Das Kondensationsmittel kann beispielsweise in Mengen von 1 bis 3 Mol, bezogen auf ein Mol der Verbindung der Formel (III), eingesetzt werden. Bevorzugt sind Kondensationsmittelmengen von 1 bis 2 Mol, bezogen auf ein Mol der Verbindung der Formel (III).

Der Reaktionsschritt a) kann beispielsweise bei 20 bis 150 °C durchgeführt werden. Bevorzugte Temperaturen sind solche von 20 bis 140 °C.

Aus dem nach dem Reaktionsschritt a) vorliegenden Reaktionsgemisch können die Verbindungen der Formel (IV) auf an sich bekannte Weise gewonnen werden, beispielsweise durch Extraktion, Destillation und/oder Kristallisation.

Im Reaktionsschritt b) werden die Verbindungen der Formel (IV) bei erhöhter Temperatur mit mindestens einer äquimolaren Menge in Phosphorpentachlorid umgesetzt. Geeignete Temperaturen sind beispielsweise solche im Bereich von 50 bis 240 °C. Vorzugsweise arbeitet man bei 60 bis 220 °C, besonders bevorzugt bei 60 bis 200 °C.

Größere Überschüsse an Phosphorpentachlorid stören im allgemeinen nicht. Aus wirtschaftlichen

Erwägungen gibt man vorzugsweise 1 bis 1,5 Mol Phosphorpentachlorid pro Mol einer Verbindung der Formel (IV) zu. Man kann das Phosphorpentachlorid auch in situ entstehen lassen, beispielsweise aus Phosphortrichlorid und elementarem Chlor.

Im allgemeinen ist es vorteilhaft, das Phosphorpentachlorid in mehreren Teilmengen nacheinander zuzusetzen. Man kann beispielsweise so verfahren, daß man zunächst pro Mol einer Verbindung der Formel (IV) nur 0,1 bis 0,3 Mol Phosphorpentachlorid zusetzt und erst nach dem Abklingen der einsetzenden Reaktion das restliche Phosphorpentachlorid zufügt. Man kann aber auch das gesamte einzusetzende Phosphorpentachlorid vorlegen und die Verbindung der Formel (IV) portionsweise zufügen.

Die Gegenwart von Lösungsmitteln ist im Reaktionsschritt b) nicht unbedingt erforderlich, in vielen Fällen ist sie jedoch vorteilhaft, z. B. zur Verbesserung der Rührbarkeit und/oder der Wärmeabfuhr. Geeignete Lösungsmittel sind beispielsweise chlorierte Kohlenwasserstoffe, insbesondere solche mit höheren Siedepunkten, wie Tetrachlorkohlenstoff, Chlorbenzol und Dichlorbenzol. Bevorzugt wird jedoch Phosphoroxychlorid als Lösungsmittel eingesetzt, da dieses auch während der Reaktion aus dem eingesetzten Phosphorpentachlorid entsteht und dann kein besonderer Aufwand für dessen Abtrennung erforderlich wird.

Die Reaktion im Reaktionsschritt b) ist bei Temperaturen von 60 bis 200 °C im allgemeinen innerhalb von 2 bis 3 Stunden abgeschlossen. Bei tieferen (höheren) Temperaturen können längere (kürzere) Reaktionszeiten vorteilhaft sein. Auch beim Einsatz von überschüssigem Phosphorpentachlorid wirken sich längere Reaktionszeiten im allgemeinen nicht negativ aus.

Die im Reaktionsschritt b) erhaltenen Verbindungen der Formel (V) können auf an sich bekannte Weise isoliert werden, beispielsweise durch Extraktion, Destillation und/oder Kristallisation. Eine Isolierung der Verbindungen der Formel (V) ist jedoch nicht zwingend erforderlich.

Im Reaktionsschritt c) werden Verbindungen der Formel (V) mit einem Fluorierungsmittel umgesetzt. Geeignete Fluorierungsmittel sind beispielsweise Fluorwasserstoff, Antimontrifluorid, Kaliumfluorid und Natriumfluorid. Vorzugsweise wird Fluorwasserstoff verwendet.

Das Fluorierungsmittel kann beispielsweise in Mengen von 2 bis 15 Mol, bezogen auf 1 Mol einer Verbindung der Formel (V) eingesetzt werden. Vorzugsweise beträgt diese Menge 3 bis 6 Mol pro Mol einer Verbindung der Formel (V).

Der Reaktionsschritt c) kann beispielsweise bei Temperaturen von —10 bis + 120 °C durchgeführt werden, wenn Fluorwasserstoff als Fluorierungsmittel eingesetzt wird. In anderen Fällen kann diese Temperatur auch höher liegen und beispielsweise bis zu 250 °C betragen. Bevorzugt sint Temperaturen von 0 bis 80 °C. Der Druck im Reaktionsschritt c) kann beispielsweise 1 bis 50 bar betragen. Vorzugsweise liegt er im Bereich von 1 bis 30 bar. Ein Lösungsmittel ist für diesen Reaktionsschritt nicht erforderlich, jedoch kann auch gegebenenfalls in Gegenwart eines Lösungsmittels gearbeitet werden. Hierfür sind beispielsweise Dichlormethan, 1,2-Dichlorethan, Chlorbenzol und Dichlorbenzol geeignet.

Die Aufarbeitung des nach dem Reaktionsschritt c) vorliegenden Reaktionsgemisches kann beispielsweise durch fraktionierte Destillation erfolgen. Nach der Abtrennung von leichtflüchtigen Bestandteilen ist insbesondere eine fraktionierte Destillation im Vakuum zur Gewinnung der Verbindungen der Formel (I) von Vorteil.

Es ist ausgesprochen überraschend, daß die neuen Verbindungen der Formel (I) auf die erfindungsgemäße Weise hergestellt werden können, denn beispielsweise ist aus J.A.C.S. 77, 1137 (1955) bekannt, daß sich zwar Ketogruppen, nicht aber Esterfunktionen (siehe Formel (IV)) mit Phosphorpentachlorid chlorieren lassen. Beispielsweise wird dort beschrieben, daß aus der Umsetzung von Acetessigsäureethylester mit Phosphorpentachlorid β-Chlorisocrotonsäureethylester, β-Chlorcrotonsäureethylester und unumgesetzter Acetessigsäureethylester erhalten wurden, nicht jedoch 1-Methoxy-1,1,3,3-tetrachlorbutan.

Die neuen Verbindungen der Formel (I) sind wertvolle Zwischenprodukte für die Herstellung von Benzoylharnstoffen, die insektizide und/oder akarizide Wirkungen haben (s. EP-A-0 173 152). Solche Benzoylharnstoffe können aus den Verbindungen der Formel (I) erhalten werden, indem man nach an sich bekannten Methoden gegebenenfalls eine Nitrogruppe einführt, die dann in jedem Fall vorhandene Nitrogruppe zu einer $NH_2$-Funktion reduziert und die so erhaltenen Amine mit Benzoylisocyanaten umsetzt.

Die folgenden Beispiele erläutern die vorliegende Erfindung ohne sie in irgendeiner Weise zu beschränken.

## Beispiele

### Beispiel 1

In 3 200 ml Wasser wurden 768 g Chloressigsäure und 880 g Brenzkatechin vorgelegt und eine Lösung von 340 g NaOH in 1,6 l Wasser zugetropft. Anschließend wurde für 4 Stunden am Rückfluß gekocht und gerührt, abgekühlt, mit Schwefelsäure sauergestellt und das Festprodukt abgesaugt. Durch Destillation im Vakuum wurden 932 g Benzodioxenon mit einem Siedepunkt von 124 bis 125 °C bei 20 mbar und einem Schmelzpunkt von 50 bis 52 °C erhalten.

Beispiel 2

In 250 ml Acetonitril wurden 70 g Kaliumcarbonat und 55 g Brenzkatechin vorgelegt und auf 50 °C erhitzt. Dann wurden 100 g Bromisobuttersäuremethylester zugetropft und für 5 Stunden am Rückfluß gekocht und gerührt. Nach dem Abdestillieren des Acetonitrils wurden zu dem verbliebenen Rückstand 200 ml Wasser gegeben, mit Salzsäure sauergestellt und mit Dichlormethan extrahiert. Durch Destillation wurden aus dem Extrakt 74 g 2,2-Dimethyl-benzodioxen-3-on mit einem Siedepunkt von 115 bis 120 °C bei 14 mbar und einem Schmelzpunkt von 42 bis 44 °C erhalten.

Beispiele 3 bis 7

Es wurde verfahren wie in Beispiel 2, jedoch wurden andere Ausgangsprodukte eingesetzt. Die Ausgangsprodukte und Reaktionsergebnisse sind aus Tabelle 1 ersichtlich.

(Siehe Tabelle 1 Seite 6 f.)

Tabelle 1

| Beispiel Nr. | Ausgangsprodukt der Formel (II) | Ausgangsprodukt der Formel (III) | Reaktionsprodukt 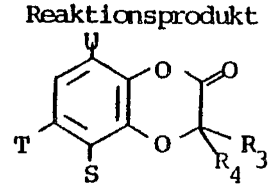 | Siedepunkt (Sdp) und Schmelzpunkt (Schmp.) des Reaktionsprodukts | Ausbeute |
|---|---|---|---|---|---|
| 3 | Brenzkatechin | $CH_3-\underset{Br}{CH}-CO_2CH_3$ | $R_3$=$CH_3$ $R_4$=H<br>S,T,U = H | Sdp: 130-135°C/20 mbar<br>Schmp: 46-48°C | 68 % |
| 4 | Brenzkatechin | Phenyl-$\underset{Br}{CH}-CO_2C_2H_5$ | $R_3$=Phenyl $R_4$=H<br>S,T,U = H | Sdp: 120-128°C/0,7 mbar<br>Schmp: 73-75°C | 82 % |
| 5 | Brenzkatechin | $C_2H_5CO_2-\underset{Cl}{CH}-CO_2C_2H_5$ | $R_3$=$CO_2C_2H_5$ $R_4$=H<br>S,T,U = H | Sdp: 124°C/18 mbar<br>Schmp: 47-49°C | 46 % |
| 6 | 4-Nitro-brenz-katechin | $CH_3-\underset{Br}{CH}-CO_2CH_3$ | $R_3$=$CH_3$ $R_4$=H<br>T = $NO_2$<br>S,U = H | Sdp: 170-178°C/0,3 mbar<br>Schmp: | 73 % |
| 7 | 3-Methyl-brenz-katechin | $CH_3-\underset{Br}{CH}-CO_2CH_3$ | 1) $R_3$,U=$CH_3$, $R_4$=H<br>S,T = H<br>2) $R_3$,S=$CH_3$, $R_4$=H<br>T,U = H<br>Gewichtsteile 1) zu 2) = 64 zu 36 | Sdp: 135-140°C/18 mbar<br>Schmp: | 78 % |

Beispiel 8

115 g 2,2-Dimethyl-benzodioxen-3-on, erhalten gemäß Beispiel 2, wurden mit 172 g Phosphorpentachlorid vermischt und unter Rühren bis zum Rückfluß erhitzt. Bei 65 °C wurde die Mischung flüssig und bei 120 °C die Rückflußtemperatur erreicht. Nach 2 Stunden wurde das entstandene Phosphoroxychlorid abdestilliert und das Rohprodukt einer Destillation unterzogen. Es wurden 128 g 2,2-Dimethyl-3,3-dichlorbenzodioxen mit einem Siedepunkt von 132 bis 140 °C bei 20 mbar und einem Schmelzpunkt von 63 bis 64 °C (umkristallisiert aus Hexan), erhalten.

Beispiele 9 bis 12

Es wurde verfahren wie in Beispiel 8, jedoch wurden andere Ausgangsprodukte eingesetzt. Die Ausgangsprodukte und die Reaktionsergebnisse sind aus Tabelle 2 ersichtlich.

(Siehe Tabelle 2 Seite 8 f.)

Tabelle 2

| Beispiel Nr. | Ausgangsprodukt der Formel (IV) erhalten gemäß Beispiel Nr. | Reaktionsprodukt 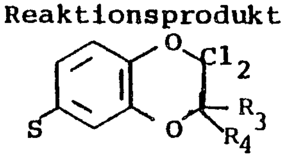 | Siedepunkt (Sdp) und Schmelzpunkt (Schmp) des Reaktionsprodukts | Ausbeute |
|---|---|---|---|---|
| 9 | 1 | $R_3$,$R_4$,S = H | Sdp: 128-134°C/18 mbar | 82 % |
| 10 | 3 | $R_3$=H, $R_4$=$CH_3$, S=H | Sdp: 125-130°C/18 mbar<br>Schmp: 46-48°C | 86 % |
| 11 | | $R_3$=H, $R_4$=H, S=$NO_2$ | Schmp: 32 - 36°C | |
| 12 | 4 | $R_3$=H, $R_4$=Phenyl, S=H | Sdp: 100-110°C/0,1 mbar | 78 % |

Beispiel 13

In einer Fluorierungsapparatur aus Edelstahl wurden 128 g 2,2-Dimethyl-3,3-dichlor-benzodioxen, erhalten gemäß Beispiel 8, vorgelegt und bei — 10 °C 250 ml Fluorwasserstoff zudosiert. Unter Rühren wurde die Temperatur langsam auf + 10 °C gesteigert und zuletzt auf 20 °C erwärmt. Es wurde gerührt bis keine Chlorwasserstoffentwicklung mehr zu beobachten war (etwa 4 Stunden). Dann wurde der überschüssige Fluorwasserstoff abdestilliert und anschließend 83 g 2,2-Dimethyl-3,3-difluor-benzodioxen mit einem Siedepunkt von 84 bis 86 °C bei 22 mbar und einem Brechungsindex von $n_D^{20}$ von 1,4740 erhalten.

Beispiel 14

In einer Edelstahlapparatur wurden 2 000 ml Fluorwasserstoff vorgelegt und bei — 3 °C 1 295 g 2,2-Dimethyl-3,3-dichlor-benzodioxen, erhalten gemäß Beispiel 8, gelöst in Dichlormethan zugetropft. Es wurde anschließend zunächst 2 Stunden auf 20 °C und dann 1 Stunde auf 60 °C unter Rühren erhitzt. Nach der Aufarbeitung des Reaktionsgemisches wie in Beispiel 13 beschrieben wurden 831 g 2,2-Dimethyl-3,3-difluor-benzodioxen erhalten.

Beispiele 15 bis 17

Es wurde verfahren wie in Beispiel 13, jedoch wurden andere Ausgangsprodukte eingesetzt. Die Ausgangsprodukte und die Reaktionsergebnisse sind aus Tabelle 3 ersichtlich.

(Siehe Tabelle 3 Seite 10 f.)

Tabelle 3

| Beispiel Nr. | Ausgangsprodukt der Formel (V) erhalten gemäß Beispiel Nr. | Reaktionsprodukt 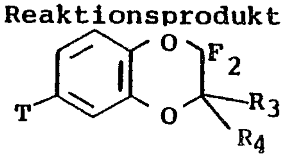 | Siedepunkt des Reaktionsprodukts | Brechungsindex $n_D^{20}$ des Reaktionsprodukts | Ausbeute |
| --- | --- | --- | --- | --- | --- |
| 15 | 10 | $R_3$=$CH_3$; $R_4$,T=H | 75-78°C/18 mbar | 1,4754 | 55 % |
| 16 | 12 | $R_3$=Phenyl; $R_4$=H; T = H | 140-145°C/8 mbar | 1,5471 | 78 % |
| 17 | | $R_4$=H; $R_3$=$CH_3$; T=$NO_2$ | 95-100°C/0,2 mbar | 1,5205 | 81 % |
| 18 | | $R_3$, $R_4$ = $CH_3$; T = $NO_2$ | 118-25°C/0,18 mbar | 1,5145 | 84 % |

**Patentansprüche**

1. Neue benzokondensierte, fluorierte, heterocyclische Verbindungen der Formel

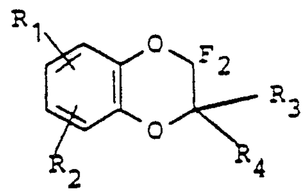

(I)

in der $R_1$ und $R_2$ unabhängig voneinander für H, F, Cl, $CH_3$, $NO_2$, CO—O—$C_1$- bis $C_4$-Alkyl, COCl oder Phenyl oder $R_1$ und $R_2$ gemeinsam für 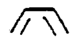 , $R_3$ für H, $C_1$- bis $C_4$-Alkyl, CO—O—$C_1$- bis $C_4$-Alkyl, CN oder Phenyl und $R_4$ für $C_1$- bis $C_4$-Alkyl oder Phenyl stehen.

2. Verfahren zur Herstellung von Verbindungen der Formel (I)

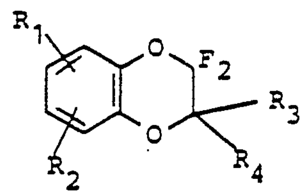

(I)

in der $R_1$ und $R_2$ unabhängig voneinander für H, F, Cl, $CH_3$, $NO_2$, CO—O—$C_1$- bis $C_4$-Alkyl, COCl oder Phenyl oder $R_1$ und $R_2$ gemeinsam für 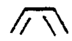 , $R_3$ für H, $C_1$- bis $C_4$-Alkyl, CO—O—$C_1$- bis $C_4$-Alkyl, CN oder Phenyl und $R_4$ für $C_1$- bis $C_4$-Alkyl oder Phenyl stehen, dadurch gekennzeichnet, daß man

a) Benzkatechin oder ein Benzkatechinderivat der Formel (II)

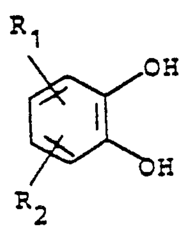

(II)

in der $R_1$ und $R_2$ die oben angegebene Bedeutung haben, mit Verbindungen der Formel (III)

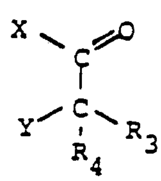

(III)

in der

$R_3$ und $R_4$ die oben angegebene Bedeutung haben,

X für Cl, Br, O—$C_1$- bis $C_4$-Alkyl, oder S—$C_1$- bis-$C_4$-Alkyl und

Y für Cl oder Br steht,

in Gegenwart eines Lösungsmittels und unter Zusatz eines Kondensationsmittels zu Verbindungen der Formel (IV),

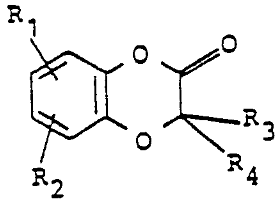

(VI)

in der $R_1$, $R_2$, $R_3$ und $R_4$ die oben angegebene Bedeutung haben, umsetzt,

b) die Verbindungen der Formel (IV) bei erhöhter Temperatur und mindestens mit einer äquimolaren Menge Phosphorpentachlorid zu Verbindungen der Formel (V)

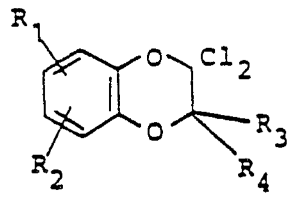

(V)

in der $R_1$, $R_2$, $R_3$ und $R_4$ die oben angegebene Bedeutung haben, umsetzt und

c) die Verbindungen der Formel (V) mit einem Fluorierungsmittel zu Verbindungen der Formel (I) umsetzt.

3. Verfahren nach Anspruch 2, dadurch gekennzeichnet, daß man im Reaktionsschritt a) als Lösungsmittel Wasser, Alkohole, Acetonitril, Dimethylformamid oder Mischungen davon und als Kondensationsmittel basische Verbindungen einsetzt, letztere in Mengen von 1 bis 3 Mol, bezogen auf ein Mol der Verbindung der Formel (III), und bei Temperaturen von 20 bis 150 °C arbeitet.

4. Verfahren nach Anspruch 2, dadurch gekennzeichnet, daß man den Reaktionsschritt b) bei 50 bis 240 °C und mit 1 bis 1,5 Mol Phosphorpentachlorid pro Mol einer Verbindung der Formel (IV) durchführt.

5. Verfahren nach Anspruch 2, dadurch gekennzeichnet, daß man im Reaktionsschritt c) 2 bis 15 Mol eines Fluorierungsmittels aus der Gruppe Fluorwasserstoff, Antimontrifluorid, Kaliumfluorid und Natriumfluorid, bezogen auf ein Mol einer Verbindung der Formel (V), einsetzt und bei — 10 bis + 120 °C arbeitet.

6. Verfahren nach Anspruch 2, dadurch gekennzeichnet, daß man nach dem Reaktionsschritt c) die Verbindungen der Formel (I) gewinnt, indem man zunächst durch fraktionierte Destillation die leichtflüchtigen Bestandteile abtrennt und danach eine fraktionierte Destillation im Vakuum durchführt.

7. Verwendung von Verbindungen der Formel (I) zur Herstellung von Benzoylharnstoffen.

**Claims**

1. New benzo-fused fluorinated heterocyclic compounds of the formula

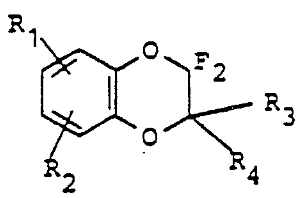

(I)

in which $R_1$ and $R_2$ independently of one another represent H, F, Cl, $CH_3$, $NO_2$, CO—O—$C_1$- to $C_4$-alkyl, COCl or phenyl or, $R_1$ and $R_2$ together represent 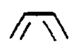 , $R_3$ represents H, $C_1$- to $C_4$-alkyl, CO—O—$C_1$ to $C_4$-alkyl, CN or phenyl and $R_4$ represents $C_1$ to $C_4$-alkyl or phenyl.

2. Process for the preparation of compounds of the formula (I)

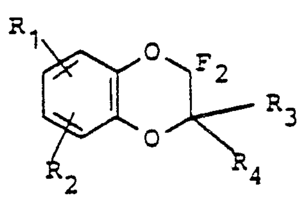

(I)

in which $R_1$ and $R_2$ independently of one another represent H, F, Cl, $CH_3$, $NO_2$, CO—O—$C_1$- to $C_4$-alkyl, COCl or phenyl, or $R_1$ and $R_2$ together represent 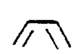 , $R_3$ represents H, $C_1$- to $C_4$-alkyl, CO—O—$C_1$- to $C_4$-alkyl, CN or phenyl and $R_4$ represents $C_1$- to $C_4$-alkyl or phenyl, characterised in that

a) pyrocatechol or a pyrocatechol derivative of the formula (II)

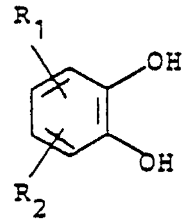

(II)

in which $R_1$ and $R_2$ have the abovementioned meaning, is reacted with compounds of the formula (III)

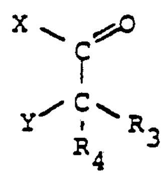

(III)

in which

$R_3$ and $R_4$ have the abovementioned meaning,

X represents Cl, Br, O—$C_1$- to $C_4$-alkyl or S—$C_1$- to $C_4$-alkyl and

Y represents Cl or Br,

in the presence of a solvent and with the addition of a condensing agent, to give compounds of the formula (IV)

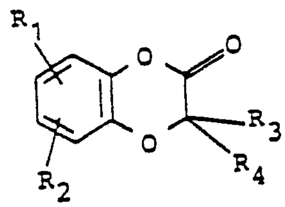

(IV)

in which $R_1$, $R_2$, $R_3$ and $R_4$ have the abovementioned meaning,

b) the compounds of the formula (IV) are reacted at elevated temperature with at least an equimolar amount of phosphorus pentachloride to give compounds of the formula (V)

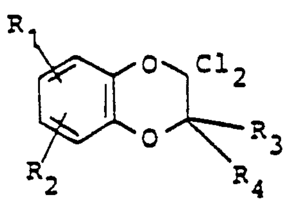

(V)

in which $R_1$, $R_2$, $R_3$ and $R_4$ have the abovementioned meaning, and

c) the compounds of the formula (V) are reacted with a fluorinating agent to give compounds of the formula (I).

3. Process according to Claim 2, characterised in that, in reaction step a), water, alcohols, acetonitrile, dimethylformamide or mixtures thereof are employed as the solvent and basic compounds are employed as the condensing agent, the latter in amounts of 1 to 3 moles per mole of a compound of the formula (III), and the reaction is carried out at temperatures from 20 to 150 °C.

4. Process according to Claim 2, characterised in that reaction step b) is carried out at 50 to 240 °C with 1 to 1.5 moles of phosphorus pentachloride per mole of a compound of the formula (IV).

5. Process according to Claim 2, characterised in that, in reaction step c), 2 to 15 moles of a fluorinating agent from the group comprising hydrogen fluoride, antimony trifluoride, potassium fluoride and sodium fluoride are employed per mole of a compound of the formula (V) and the reaction is carried out at — 10 to + 120 °C.

6. Process according to Claim 2, characterised in that, after reaction step c), the compounds of the formula (I) are isolated by first removing the highly volatile constituents by fractional distillation and then carrying out fractional distillation in vacuo.

7. Use of compounds of the formula (I) for the preparation of benzoylureas.

**Revendications**

1. Nouveaux composés hétérocycliques fluorés condensés au benzène, de formule,

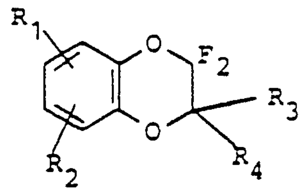

(I)

dans laquelle $R_1$ et $R_2$ représentent, indépendamment l'un de l'autre, H, F, Cl, $CH_3$, $NO_2$, un groupe CO—O-(alkyle en $C_1$ à $C_4$), COCl ou phényle ou bien $R_1$ et $R_2$ forment conjointement un groupe 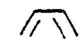 , $R_3$ représente H, un groupe alkyle en $C_1$ à $C_4$, un groupe CO—O-(alkyle en $C_1$ à $C_4$), CN ou phényle et $R_4$ est un groupe alkyle en $C_1$ à $C_4$ ou phényle.

2. Procédé de production de composés de formule (I)

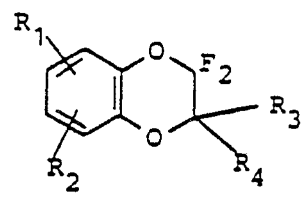

(I)

dans laquelle $R_1$ et $R_2$ représentent, indépendamment l'un de l'autre, H, F, Cl, $CH_3$, $NO_2$, un groupe CO—O-(alkyle en $C_1$ à $C_4$), COCl ou phényle ou bien $R_1$ et $R_2$ forment conjointement un groupe 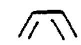 , $R_3$ représente H, un groupe alkyle en $C_1$ à $C_4$, un groupe CO—O-(alkyle en $C_1$ à $C_4$), CN ou phényle et $R_4$ est un groupe alkyle en $C_1$ à $C_4$ ou phényle, caractérisés en ce que :

a) on fait réagir du pyrocatéchol ou un dérivé de pyrocatéchol de formule (II)

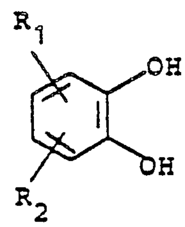

(II)

dans laquelle $R_1$ et $R_2$ ont la définition indiquée ci-dessus, avec des composés de formule (III)

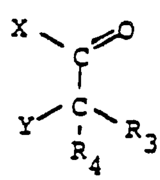

(III)

dans laquelle

$R_3$ et $R_4$ ont la définition indiquée ci-dessus,

X représente Cl, Br, un groupe O-(alkyle en $C_1$ à $C_4$) ou S-(alkyle en $C_1$ à $C_4$) et

Y représente Cl ou Br,

en présence d'un solvant et avec addition d'un agent de condensation pour former des composés de formule (IV)

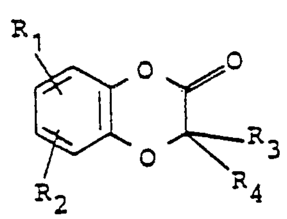

(IV)

dans laquelle $R_1$, $R_2$, $R_3$ et $R_4$ ont la définition indiquée ci-dessus,

b) on fait réagir les composés de formule (IV) à température élevée et avec au moins une quantité équimolaire de pentachlorure de phosphore pour former des composés de formule (V)

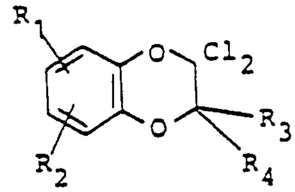

(V)

dans laquelle $R_1$, $R_2$, $R_3$ et $R_4$ ont la définition indiquée ci-dessus, et

c) on fait réagir les composés de formule (V) avec un agent de fluoration pour obtenir des composés de formule (I).

3. Procédé suivant la revendication 2, caractérisé en ce qu'on utilise comme solvants dans l'étape de réaction a) l'eau, des alcools, l'acétonitrile, le diméthylformamide ou leurs mélanges et comme agents de condensation, des composés basiques, ces derniers en quantités de 1 à 3 moles pour une mole de composé de formule (III) et on opère à des températures de 20 à 150 °C.

4. Procédé suivant la revendication 2, caractérisé en ce qu'on conduit l'étape réactionnelle b) à une température de 50 à 240 °C et avec 1 à 1,5 mole de pentachlorure de phosphore par mole d'un composé de formule (IV).

5. Procédé suivant la revendication 2, caractérisé en ce qu'on utilise dans l'étape de réaction c) 2 à 15 moles d'un agent de fluoration du groupe du fluorure d'hydrogène, du trifluorure d'antimoine, du fluorure de potassium et du fluorure de sodium, par rapport à une mole d'un composé de formule (V), et on opère entre — 10 et + 120 °C.

6. Procédé suivant la revendication 2, caractérisé en ce que, après l'étape de réaction c), on obtient les composés de formule (I) en séparant tout d'abord par distillation fractionnée les constituants très volatils, puis en conduisant une distillation fractionnée sous vide.

7. Utilisation des composés de formule (I) pour la préparation de benzoylurées.